# FASCICULE DE BREVET EUROPEEN

(11) **EP 2 529 725 B1**
(45) Date de publication et mention de la délivrance du brevet: **14.03.2018**
(21) Numéro de dépôt: 12169562.1
(22) Date de dépôt: 25.05.2012
(51) Int. Cl.: A61K 8/25, A61K 8/31, A61K 8/34, A61K 8/90, A61Q 1/10, A61Q 1/12

(54) **Composition cosmétique pour le soin ou le maquillage contenant des poudres et procédé de fabrication**
Kosmetische Pflege- oder Schminkzusammensetzung, die Puder enthält, und entsprechendes Herstellungsverfahren
Cosmetic care or make-up composition containing powders and manufacturing method

(30) Priorité: 31.05.2011 FR 1154739
(43) Date de publication de la demande: 05.12.2012
(73) Titulaire: LVMH RECHERCHE, 45800 St. Jean de Braye (FR)
(72) Inventeur: Vic, Sabine, 45400 SEMOY (FR); Ferrand, Philippe, 45430 CHECY (FR); Perrier, Eric, 38138 LES COTES D'AREY (FR)
(74) Mandataire: Chantraine, Sylvie Hélène

(56) Documents cités:
- EP-A1- 0 968 705
- EP-A1- 1 116 733
- EP-A1- 1 216 693
- EP-A1- 1 977 729
- EP-A2- 0 165 137
- EP-A2- 1 776 986
- WO-A1-03/022236
- WO-A1-2005/070384
- DE-A1-102009 037 934
- FR-A1- 2 779 960
- FR-A1- 2 873 583
- US-A1- 2002 011 186
- US-A1- 2005 037 038
- US-A1- 2005 249 684
- US-A1- 2008 138 302
- US-A1- 2009 035 243

## Description

L'invention concerne une composition à base de poudres, particulièrement destinée au soin cosmétique et/ou au maquillage de la peau. Cette composition contient une phase solide particulaire à base de charges et de pigments, une phase liquide non volatile et un polymère particulier.

### ETAT DE L'ART

Les produits cosmétiques à base de poudres pour le maquillage et/ou le soin du visage et du corps peuvent être des poudres libres ou des poudres compactes.

Les compositions cosmétiques à base de poudres qu'elles soient libres ou compactes peuvent être préparées par voie « sèche » ou par voie « humide » si l'on utilise un solvant ou un diluant pour disperser les poudres. Par voie sèche, les matériaux pulvérulents tels que les charges et les pigments sont mélangés avec un liant essentiellement composé de corps gras non volatils. Par voie humide, les matériaux pulvérulents et le liant sont dilués dans un solvant pour obtenir une pâte (appelée « slurry » en anglais) qui est ensuite séchée.

Une poudre libre est généralement préparée à sec par simple mélange des ingrédients, éventuellement accompagné d'un broyage et d'un tamisage.

Les poudres compactes sont généralement préparées en mélangeant l'ensemble des composants de la phase pulvérulente puis en ajoutant à ce mélange une phase grasse liante sous agitation. Le mélange est ensuite broyé, tamisé, puis versé dans une coupelle et compacté. Le compactage est typiquement réalisé à une pression de 5 à 25 MPa.

Une poudre compacte est différente d'une composition « coulée », qui est préparée par chauffage d'une phase grasse solide à température ambiante et qu'il est nécessaire de fondre pour être mélangée à des matériaux pulvérulents. Les poudres compactes ont, comparativement aux compositions coulées, une meilleure aptitude au délitage lors du prélèvement de la composition en vue de son application sur la peau.

Il existe aussi des poudres compactes fabriquées par voie humide dont le procédé de fabrication met en oeuvre une pâte (slurry) et ne comprend pas une étape de compactage à sec.

Par exemple, le procédé dit « coulé-cuit » consiste à diluer les poudres dans un solvant à température ambiante pour préparer un pâte, à déposer des morceaux de la pâte obtenus sur une plaque de céramique, puis à cuire le produit en étuve. Les cakes de poudre séchés sont ensuite fraisés à la forme du boîtier qui doit servir de conditionnement. Le support céramique auquel le cake de poudre adhère reste solidaire de la composition avec laquelle il est donc conditionné.

Un autre procédé consiste à couler la pâte dans des moules, puis à éliminer le solvant contenu dans la pâte par exemple par aspiration, par pressage ou à l'aide d'un matériau absorbant.

Il est souvent difficile d'obtenir des poudres cosmétiques de bonne qualité car de nombreux critères - tels que leurs propriétés sensorielles, leurs performances de soin ou de maquillage, et leur cohésion - sont à prendre en compte. Au surplus, l'amélioration de certains critères se fait souvent au dépend des autres. Par exemple, un meilleur délitement conduit souvent à un manque de cohésion de la poudre. Un meilleur crémeux conduit souvent à un compact moins pratique pour l'utilisateur à cause d'un risque important de mauvais prélèvement en surface, celle-ci ayant une forte tendance à cirer ou patiner.

Les propriétés recherchées pour obtenir une poudre cosmétique de bonne qualité incluent notamment le pouvoir couvrant, l'adhérence, le pouvoir d'absorption de l'humidité et du sébum de la peau, le pouvoir d'étalement, la douceur, la finesse, le confort sur la peau, une longue tenue sur la peau, l'homogénéité, une stabilité à la conservation, et un prélèvement aisé de la poudre en vue de son application.

Il est en outre nécessaire d'obtenir une bonne cohésion de la poudre, qui peut se traduire par exemple par une bonne résistance aux chocs mécaniques et aux chocs thermiques des poudres lorsqu'elles sont compactes, ou par une volatilité moindre à l'usage et/ou une meilleure tenue sur la peau des poudres lorsqu'elles sont libres.

La résistance aux chocs mécaniques et/ou aux chocs thermiques des poudres compactes est souvent insuffisante, ce qui génère la casse du produit ou une dégradation du produit lors du transport jusqu'au lieu de vente. L'utilisateur du produit peut également par inadvertance faire tomber le produit d'une hauteur significative.

Des poudres compactes dotées de bonnes propriétés sensorielles, d'une bonne stabilité et de bonnes propriétés de cohésion ont déjà été décrites dans le document EP 968 705.

Par ailleurs, les propriétés de gélification de l'eau d'un copolymère de l'acide acrylamido-2-méthylpropane-sulfonique ont également largement décrites dans les documents WO 2005/070384, EP 1 116 733 et WO 03/022236. Le fard à paupières divulgué dans le document EP 1 216 693 est sous forme d'un gel aqueux contenant le même copolymère. Ce produit de maquillage est doté de nuances de couleurs plus fortes, de meilleures propriétés sensorielles, tels que l'étalement sur la peau ou l'absorption dans la peau, et d'une plus grande stabilité.

Dans le document EP 1 776 986, un copolymère de l'acide acrylamido-2-méthylpropane-sùlfonique est utilisé pour une autre fonction : il sert d'agent tensio-actif dans des crèmes solaires en émulsion huile-dans-eau. Dans les exemples des documents EP 1 977 729 et DE 10 2009 037 934, un copolymère de l'acide acrylamido-2-méthylpropane-sulfonique est présent dans la phase aqueuse d'un fond de teint en émulsion, sans que sa fonction ne soit cependant précisée.

Il est également connu que ce copolymère épaissit les solvants polaires non aqueux tels que les glycols, les polyalkylène glycols et les sucres (voir US 2009/0035243). Enfin, il peut entrer dans la composition d'un gel de polissage utilisé pour réaliser une dermabrasion. Il a alors pour fonction la protection de la peau pour améliorer le glissant du soin lors de l'application (ceci est décrit dans le document US 2005/0037038).

Des procédés de fabrication de poudres compactes qui mettent en oeuvre des solvants que l'on mélange aux poudres pour pouvoir les mouler sont par ailleurs connus. Le solvant utilisé est par exemple une silicone volatile (FR 2 779 960) ou le chlorofluorocarbone (EP 165 137).

### BUTS DE L'INVENTION

La présente invention vise à proposer de nouvelles compositions cosmétiques à base de poudres améliorant les propriétés des poudres de l'art antérieur, et améliorant notamment la résistance aux chocs mécaniques et thermiques, le confort de la poudre sur la peau, la tenue de la poudre sur la peau, ou l'ensemble de ces propriétés à la fois.

L'invention a également pour but de résoudre le problème technique consistant à fournir une poudre compacte qui soit homogène.

L'invention a également pour but de simplifier les procédés industriels actuels en proposant une composition unique permettant une plus grande flexibilité de fabrication. Cette composition permet notamment d'obtenir différentes formes finales de poudres et ainsi de réduire le nombre de lignes de production.

L'invention a pour but de résoudre les problèmes techniques de manière fiable, reproductible et utilisable à l'échelle industrielle, en particulier en cosmétique.

### DESCRIPTION DE L'INVENTION

Ainsi, la présente invention concerne une composition cosmétique sous forme solide, notamment sous forme de poudre libre ou compacte, comprenant :
(a) de 70 à 99% en poids d'au moins une phase solide particulaire,
(b) de 0,1 à 50% en poids, de préférence de 5 à 30% en poids, d'au moins une phase liquide non volatile, et
(c) de 0,01 à 4% en poids, de préférence de 0,05 à 2% en poids, d'au moins un copolymère bloc obtenu à partir d'un premier monomère acrylamide portant un groupe sulfonique et d'un deuxième monomère vinylique ayant une chaine latérale azotée.

La composition selon l'invention est dotée de l'un au moins des avantages suivants par rapport aux poudres de l'art antérieur: son aspect brillant est plus attractif à la vente, ses propriétés sensorielles sont améliorées, sa stabilité lors du stockage est meilleure, sa cohésion au cours de sa manipulation est améliorée, ses performances de maquillage sont améliorées. Dans certains modes de mise en oeuvre, tous ces avantages peuvent être obtenus à la fois.

Plus précisément, la composition selon l'invention peut présenter l'avantage d'être moins fragile et plus stable que les poudres de l'art antérieur lorsqu'elle est prélevée par l'utilisateur ou lorsqu'elle est soumise à des chocs. La demanderesse a trouvé de manière inattendue que le copolymère bloc permet de renforcer la cohésion et la stabilité de la composition.

Les propriétés sensorielles de la composition de l'invention peuvent également être améliorées. En particulier, dans le cas de compositions comprenant des nacres, la surface de la composition telle qu'elle est observée par un utilisateur est plus brillante que la surface des poudres de l'art antérieur.

Les liants gras utilisés pour agglomérer les poudres ont tendance à matifier la surface d'un produit compacté. Or, de façon tout à fait inattendue, la demanderesse a observé que la composition de l'invention peut contenir des quantités de liants gras très élevées sans que la texture ne prenne un aspect mat ou cireux. Le fait d'augmenter la quantité de liants gras permet d'augmenter la sensation de douceur, voir même de crémeux, de la composition lorsqu'on la prélève au doigt ou qu'on l'applique sur la peau. La composition de l'invention a donc un aspect plus brillant que les poudres de l'art antérieur dotées d'un crémeux équivalent.

La demanderesse a également trouvé que la composition peut contenir des quantités de liant gras inférieures à celles habituellement nécessaires pour garantir la cohésion de la poudre. Dans certains modes de réalisation la quantité de liant gras est inférieure à 0,1% en poids.

La composition appliquée sur la peau, par exemple les paupières ou les joues, peut procurer une sensation de confort supérieure à celles des poudres de l'art antérieur.

La tenue de la composition sur la peau peut également être améliorée. En particulier les nacres et les paillettes qui sont contenues dans la composition restent sur la peau plus longtemps. Cette tenue améliorée peut se traduire par une meilleure tenue aux frottements et/ou une meilleure résistance à l'eau. L'amélioration de la tenue est particulièrement appréciable et avantageuse pour la formulation de compositions appliquées sur les paupières comme les fards à paupières, parce qu'elles subissent des frottements répétés du fait de la mobilité naturelle des paupières.

La composition contient un copolymère bloc obtenu à partir d'un premier monomère acrylamide portant un groupe sulfonique et d'un deuxième monomère vinylique ayant une chaine latérale azotée.

Selon un mode de mise en oeuvre, le copolymère bloc gélifie en présence d'eau. Il a de préférence une viscosité mesurée à 1% dans de l'eau distillée comprise entre 40 000 et 70 000 mPa.s.

Le premier monomère est de préférence un sel de l'acide acrylamido-2-méthylpropane-sulfonique. Le groupe acide sulfonique du premier monomère est de préférence sous la forme d'un sel d'ammonium ou de sodium.

Le deuxième monomère est de préférence non salifiable. Il peut être un monomère vinylique ayant une chaîne latérale cyclique azotée ou un monomère vinylique ayant une fonction amide, comme par exemple la N-vinylpyrrolidone.

Un copolymère bloc préféré peut être un copolymère du sel d'ammonium de l'acide acrylamido-2-méthylpropane-sulfonique et de N-vinylpyrrolidone, comme celui commercialisé sous le nom Aristoflex® AVC par la société Clariant. Le copolymère bloc peut porter le nom INCI Ammonium Acryloyldimethyltaurate / VP Copolymer ou correspondre au numéro de référence CAS 335383-60-3. Un autre copolymère bloc utilisable dans la composition de l'invention peut être un copolymère du sel de sodium de l'acide acrylamido-2-méthylpropane-sulfonique et de N-vinylpyrrolidone, comme celui commercialisé sous le nom Aristoflex® AVS par la société Clariant. Le copolymère bloc peut porter le nom INCI Sodium Acryloyldimethyltaurate / VP Crosspolymer.

La composition contient de préférence de 0,05 à 2% en poids, de préférence encore de 0,1 à 0,4% en poids du copolymère bloc.

La phase solide particulaire est avantageusement constituée d'un mélange de matériaux pulvérulents comprenant au moins un pigment et/ou au moins une charge.

Les pigments peuvent être choisis parmi les pigments minéraux, les pigments organiques et les pigments nacrés.

Les pigments minéraux peuvent être choisis parmi les oxydes de fer, en particulier les oxydes de fer noir, jaune, rouge et brun; le violet de manganèse; le bleu outremer; les oxydes de chrome, en particulier l'oxyde de chrome hydraté, le bleu ferrique, le noir de carbone, et leurs mélanges.

Parmi les pigments organiques, on peut citer notamment les laques obtenues à partir de colorants tels que les colorants D&C Black No. 2, FD&C Blue No. 1, FD&C Green No. 3, D&C Green No. 5, D&C Orange No. 4, D&C Orange No. 5, D&C orange No. 10, D&C No. red 3, D&C Red No. 6, D&C Red No. 7, D&C red No. 9, D&C red No. 13, D&C red No. 19, D&C Red No. 21, D&C Red No. 22, D&C Red No. 27, D&C Red No. 28, D&C Red No. 30, D&C Red No. 33, D&C Red No. 36, FD&C Red No. 40, FD&C Yellow No. 5, FD&C Yellow No. 6, D&C Yellow No. 10 et le carmin de cochenille.

Les pigments nacrés sont par exemple choisis parmi le mica recouvert d'oxyde de titane, le mica-titane recouvert d'oxyde de fer, le mica-titane recouvert de bleu ferrique, le mica-titane recouvert d'oxyde de chrome, le mica-titane recouvert d'un pigment organique tel que décrit précédemment, ainsi que les pigments à base d'oxychlorure de bismuth.

Les charges peuvent être minérales ou organiques, et de toutes formes, plaquettaires, sphériques ou oblongues.

Les charges sont choisies notamment parmi des charges inorganiques telles que:
- le talc de préférence sous forme de particules généralement de dimensions inférieures à 40 µm;
- les micas d'origine naturelle ou synthétique ayant des dimensions de 2 à 200 µm, de préférence de 5 à 70 µm et une épaisseur de 0,1 à 5 µm, de préférence de 0,2 à 3 µm;
- le kaolin ayant des tailles de particules généralement inférieures à 30 µm;
- les savons métalliques dérivés d'acides organiques carboxyliques ayant de 8 à 22 atomes de carbone, de préférence de 12 à 18 atomes de carbone, par exemple le stéarate de zinc, le stéarate de magnésium ou le stéarate de lithium, le laurate de zinc, le myristate de magnésium, de préférence sous la forme de particules ayant des dimensions inférieures à 10 µm;
- les oxydes de zinc, les oxydes de titane; le carbonate de calcium; le carbonate de magnésium, l'hydrocarbonate de magnésium, la silice, les billes de verre, les billes de céramique;
- et leurs mélanges.

On peut aussi utiliser comme charges des charges organiques telles que:
- des amidons réticulés ou non, par exemple des amidons de maïs, de blé, ou de riz;
- des poudres de polymères de synthèse, réticulées ou non, sphéronisées ou non, expansées ou non, comme des poudres de polyéthylène, des poudres de polyester (par exemple isophtalate ou téréphtalate), des poudres de polyamide (par exemple des poudres de poly-β-alanine et des poudres de nylon comme celles commercialisées sous la dénomination ORGASOL®), des poudres d'acide poly(méth)acrylique ou de poly(méth)acylate telles que les poudres de méthacrylate de méthyle réticulé, des poudres de polyuréthane telles que les copolymères de diisocyanate d'hexaméthylène et de triméthylol hexyl lactone vendus sous les dénominations PLASTIC POWDER® D-400 et PLASTIC POWDER® D-800 par la société TOSHIKI, des poudres de polystyrène réticulé par le divinylbenzène, des poudres de résine de silicone telles que des silsesquioxanes, ou des poudres de tétrafluoroéthylène (Téflon®) ;
- et leurs mélanges.

De façon avantageuse, la composition de l'invention peut contenir de grandes proportions de poudres sphériques tout en gardant une cohésion satisfaisante ce qui n'est pas le cas des poudres de l'art antérieur. En effet, il est généralement nécessaire d'appliquer des forces de compression élevées pour faire adhérer les particules de poudres sphériques entre elles car elles ont tendance à rouler et à glisser les unes par rapport aux autres. Le copolymère utilisé dans la composition selon l'invention permet de rendre ces particules cohésives sans devoir les compacter à une pression élevée.

Selon un mode de réalisation, la composition de l'invention contient des charges sphériques en une teneur allant de 10 % à 40 % en poids, par rapport au poids total de la composition, de préférence allant de 15 % à 35 % en poids, préférentiellement allant de 25 % à 35 % en poids.

Les charges ont avantageusement un diamètre moyen supérieur à 100 nm et inférieur à 200 µm, et plus particulièrement un diamètre compris entre 5 à 150 µm.

Selon une variante de mise en oeuvre de l'invention, les pigments et les charges peuvent être traitées de façon à en modifier leur état de surface. Les pigments et les charges utilisés peuvent être avantageusement dépourvus de traitement de surface pour les rendre hydrophobes ou hydrophiles, tout en garantissant une cohésion de la poudre de l'invention suffisante.

La composition de l'invention contient au moins une phase liquide non volatile.

Par « phase liquide non volatile », on entend une phase comprenant un liquide non volatil, c'est-à-dire un liquide présentant une pression de vapeur inférieure à 13 Pa à pression atmosphérique (environ 0,1 MPa) et à température ambiante (25°C). On peut également définir un liquide non volatil comme ayant une vitesse d'évaporation telle que la quantité évaporée au bout de 30 minutes est inférieure à 0,07 mg/cm² dans les conditions de température et de pression définies précédemment.

Dans un mode de réalisation, cette phase liquide non volatile contient au moins une huile non volatile. Cette huile est de préférence le composant majoritaire en poids de ladite phase. Dans un autre mode de réalisation, la phase liquide est exempte d'huile et contient majoritairement un alcool non volatil.

Au sens de la présente invention, on entend par « huile » un composé liquide à température ambiante (25°C) et à pression atmosphérique (environ 0,1 MPa) qui n'est pas soluble dans l'eau à 25°C à une concentration d'au moins 1% en poids par rapport au poids d'eau.

L'huile non volatile peut être choisie parmi celles habituellement utilisées en cosmétique et particulièrement parmi les hydrocarbures linéaires ou ramifiés, les acides gras éventuellement ramifiés et/ou insaturés, les alcools gras éventuellement ramifiés et/ou insaturés, les esters ou polyesters d'acides gras et/ou d'alcools gras, les huiles perfluorées et/ou organofluorées, les huiles de silicone non volatiles, les huiles fluorosiliconées et leurs mélanges.

La phase liquide non volatile peut également contenir un alcool non volatil choisi parmi :
- les polyols en C3-C5, par exemple un diol aliphatique (ou glycol) en C3-C5 tel que le pentylène glycol (pentane-1,5-diol), ou un triol tel que le glycérol,
- un éther de glycol ou un alcool aromatique comme le phénoxyéthanol, et
- leurs mélanges.

La phase liquide non volatile contient de préférence au moins un liquide choisi parmi une huile, un alcool choisi parmi les polyols C3-C5, notamment le glycérol, les éthers de glycol, et leurs mélanges. Selon un mode de mise en oeuvre, la phase liquide non volatile contient moins de 0,1% d'huile ou est exempte d'huile.

La composition de l'invention peut éventuellement comprendre des agents structurants ou gélifiants de la phase liquide non volatile, notamment des corps gras pâteux ou des cires, d'origine animale, végétale, minérale ou synthétique, les polyorganosiloxanes, les polyamides siliconés comprenant des motifs siloxane, les polyamides hydrocarbonés, les homopolymères et les copolymères comprenant des unités uréthane et/ou urée, les homopolymères et copolymères bloc comprenant au moins un enchaînement d'unités styrène ou dérivés et au moins un enchaînement d'unités oléfine et/ou dérivés.

La composition à base de poudres peut avantageusement incorporer tout type d'actifs cosmétiques qu'ils soient hydrophiles ou lipophiles. Les agents actifs préférés sont choisis parmi le groupe constitué des substances ayant une activité anti-vieillissement; des substances ayant une activité dépigmentante ou une activité éclaircissante de la peau; des substances ayant une activité amincissante; des substances ayant une activité hydratante; des substances ayant une activité calmante, apaisante ou relaxante ; et de leurs mélanges.

La composition peut en outre comprendre au moins un excipient choisi parmi des colorants, des agents tensioactifs, des parfums, des électrolytes, des agents anti-oxydants, des conservateurs, et des filtres UV physiques et/ou chimiques.

La composition est avantageusement dépourvue d'agent tensio-actif, car il n'est pas nécessaire contrairement aux poudres de l'art antérieur obtenue par l'empâtage des matières pulvérulentes avec de l'eau, d'utiliser un agent tensio-actif pour garantir une bonne homogénéité de la pâte. Sans que ce mode de réalisation soit exclu, on préfère que la composition de l'invention ne contiennent pas un agent tensio-actif choisi parmi les esters de sorbitane éventuellement polyéthoxylés, les esters d'acides gras et de glycérol, les esters ou polyesters d'acides gras et de sucrose, les esters d'acides gras et de polyétrylèneglycol, les polysiloxanes modifiés polyéthers, les éthers d'alcools gras et de polyéthylèneglycol, les alkylpolyglycosides et la lécithine hydrogénée.

La composition de l'invention peut avoir divers usages cosmétiques, et notamment servir comme fond de teint, base de teint, poudre de maquillage du corps, poudre de soin, poudre de maquillage du visage, blush ou fard à joues, fard à paupières, enlumineur de teint, poudre de soleil, ou poudre de teint protectrice. La composition de l'invention est de préférence un fard à paupière ou un fard à joues.

L'application des poudres peut être réalisée au pinceau, à l'éponge ou au doigt.

L'utilisation d'un copolymère bloc tel que défini précédemment permet d'améliorer la cohésion d'une poudre. Dans le cas d'une poudre compacte, l'amélioration de sa cohésion peut se traduire par une meilleure résistance aux chocs mécaniques et/ou une meilleure résistance aux chocs thermiques. Dans le cas d'une poudre libre, l'amélioration de sa cohésion peut se traduire par une volatilité plus faible et une meilleure tenue sur la peau. Une poudre sera « volatile » lorsqu'elle aura tendance à se répandre dans l'air au moment de la prélever avec un pinceau ou au moment de l'appliquer sur la peau. L'amélioration de la cohésion de la poudre libre pourra également se traduire par une meilleure accroche des matériaux pulvérulents constituant la poudre, tels que les paillettes, qui ont parfois tendance à se détacher de la peau au cours du temps après l'application de la composition. Le copolymère bloc tel que défini précédemment permet également d'améliorer la tenue d'une composition à base de poudres sur la peau.

La composition de l'invention à base de poudres peut être réalisée par voie humide en préparant une pâte (ou composition pâteuse) selon un procédé industriel adéquat. Elle peut également être réalisée par voie sèche.

Selon un de ses aspects, l'invention a pour objet un procédé de fabrication de la composition à base de poudres décrite précédemment, lequel procédé comprend :
(i) le mélange de la phase solide particulaire, de la phase liquide non volatile, du copolymère bloc et d'un liquide volatil pour obtenir une pâte,
(ii) la mise en moule, et
(iii) l'élimination dudit liquide volatil présent dans la pâte.

Le procédé de la présente invention permet avantageusement d'obtenir une pâte homogène et des compositions sèches homogènes. On entend par « homogène » une composition dont les différents constituants sont répartis pour donner un aspect uniforme ou sensiblement uniforme à l'oeil nu. Une composition sous forme de pâte est homogène par exemple lorsqu'elle ne comprend une seule phase, ne comprend pas de grumeaux, c'est-à-dire de particules solides ou d'agglomérats solides de matériaux mal dispersés. Il n'est pas nécessaire de broyer la pâte pour obtenir une bonne dispersion des ingrédients, contrairement à certains procédés de l'art antérieur

La composition pâteuse est très avantageuse puisqu'elle peut être utilisée pour fabriquer une composition à base de poudres, qu'elle soit la forme finale désirée : compacte ou libre.

Le procédé selon l'invention génère moins de gâche industrielle car il n'est pas nécessaire de fraiser les cakes de poudres comme dans un procédé « coulé-cuit ».

La composition pâteuse contenant le mélange de la phase solide particulaire, de la phase liquide non volatile, du copolymère bloc et du liquide volatil peut être avantageusement utilisée dans tout procédé classique de préparation de poudres par voie humide connus de l'homme du métier.

Selon un autre de ses aspects, l'invention a pour objet un procédé de fabrication d'une composition à base de poudres, lequel procédé comprend :
(i) la mise en dispersion dans l'eau d'une phase solide particulaire, d'une phase liquide non volatile, et d'un copolymère bloc ionique obtenu à partir d'un premier monomère d'un sel de l'acide acrylamido-2-alkyl-sulfonique et d'un deuxième monomère, pour obtenir une pâte,
(ii) la mise en moule, et
(iii) l'élimination de l'eau présente dans la pâte par aspiration sous vide suivie d'un séchage à une température inférieure à 70°C.

Le premier monomère peut être l'acide acrylamido-2-méthylpropane-sulfonique ou un de ses sels, comme un sel d'ammonium ou de sodium.

Le deuxième monomère peut être choisi parmi l'acide (méth)acrylique, les (méth)acrylate, les (méth)acrylamides, les sels de (méth)acrylate, la vinylpyrolidone, et leurs dérivés.

Le copolymère bloc peut être par exemple un copolymère bloc d'un sel d'acide acrylamido-2-méthylpropane-sulfonique et de N-vinylpyrrolidone comme celui commercialisé sous le nom Aristoflex® AVC ou le nom Aristoflex® AVS par la société Clariant, un copolymère d'un sel d'acide acrylamido-2-méthylpropane-sulfonique et d'un sel d'acrylate comme les produits commercialisés sous les références Simulgel EG® et Creagel EZ®.

Le copolymère peut avoir l'un des noms INCI suivants :
- Sodium Acrylate / Sodium Acryloyldimethyltaurate Copolymer,
- Sodium Acryloyldimethyltaurate / VP Crosspolymer,
- Acrylamide / Sodium Acryloyldimethyltaurate Copolymer, ou
- Ammonium Acryloyldimethyltaurate / VP Copolymer.

La pâte est obtenue par mélange de la phase solide particulaire, de la phase liquide non volatile, du copolymère bloc et d'un liquide volatil. On entend par liquide volatil, un liquide qui présente une température de vaporisation inférieure ou égale à 110°C à une pression atmosphérique (environ 0,1 MPa ou 1 bar). On peut également définir un liquide volatil comme un liquide ayant une pression de vapeur supérieure à 13 Pa à pression atmosphérique (environ 0,1 MPa) et à température ambiante (25°C). On peut encore définir un liquide volatil comme un liquide ayant une vitesse d'évaporation telle que, la quantité évaporée au bout de 30 minutes est supérieure à 0,07 mg/cm² dans les conditions de température et de pression définies précédemment.

Avantageusement, le liquide volatil est choisi parmi : l'eau ; un alcool en C2-C5 de préférence choisi parmi les mono-alcools aliphatiques; un mélange eau-alcool en C2-C5 ; un diol aliphatique (ou glycol) en C3-C6 tel que le propane-1,2-diol (ou propylène glycol), le propane-1,3-diol, un butylène glycol, un polyéthylène glycol, ou le dipropylène glycol ; une isoparaffine telle que l'isododécane ou l'isohexadécane; une huile siliconée cyclique telle que la tétra-, penta-, ou hexa-cyclométhicone, ou une huile siliconée linéaire telle qu'une diméthicone possédant par exemple une viscosité de 0,65 à 5 centistokes ; les alcanes fluorés en C1-C5 ; les huiles fluorosiliconées telles que les fluoro-alkyl dimethylsiloxanes ; et l'un quelconque de leurs mélanges.

Selon un mode de réalisation, le liquide volatil contient un dispersant du copolymère bloc.
Selon un aspect de l'invention, le liquide volatil contient de l'eau, de préférence en une quantité suffisante pour faire gélifier le copolymère bloc. Selon un mode de réalisation, le liquide volatil est l'eau ou un mélange eau-alcool en C2-C5, et le copolymère est un copolymère bloc d'un sel d'acide acrylamido-2-méthylpropane-sulfonique et de N-vinylpyrrolidone.

La pâte comprend les différents constituants de préférence dans les proportions suivantes:
- de 15% à 80% en poids par rapport au poids total de la pâte de la phase solide particulaire ;
- 20 à 70% en poids par rapport au poids total de la pâte du liquide volatil;
- 0,1 à 30% en poids par rapport au poids total de la pâte de phase liquide non volatile,
- 0,01 à 2% en poids par rapport au poids total de la pâte de copolymère bloc.

Le liquide volatil représente de préférence de 25% à 60% en poids, de préférence de 25 à 35% en poids de la composition pâteuse.

Le liquide volatil, la phase liquide non volatile et le copolymère sont de préférence mélangés dans une première étape, à une température de l'ordre de 25°C, sous agitation dans un mélangeur connu de l'homme de l'art, avant d'ajouter les charges et les pigments.

La pâte peut être placée dans des moules afin de subir une mise en forme selon une technique connue de l'homme du métier.

Les moules peuvent être en métal ou en silicone et être réutilisable une fois que la composition a été démoulée. Les moules peuvent aussi être des coupelles ou des godets qui restent solidaires de la composition une fois fabriquée pour être ensuite placés dans le logement d'un conditionnement.

Lorsque la pâte est liquide, elle peut être injectée par le fond du moule ou être coulée par simple remplissage du moule par son ouverture supérieure. Un procédé d'injection par le fond des moules est par exemple connu sous le nom « Back Injection Machine » ou BIM. Un procédé de remplissage des moules qui consiste à verser la pâte dans le moule par le dessus est connu sous le nom de « Top Fill ».
La pâte peut également être plus visqueuse si bien que l'on peut la doser en préparant des morceaux de pâte. Le pourcentage de liquide volatil est
de préférence choisi de telle sorte que la pâte possède une consistance suffisante pour pouvoir être pétrie, dosée et découpée. Dans ce cas, il est nécessaire de mettre en forme la pâte pour que la composition épouse les bords de godets, avant d'éliminer le liquide volatil. Cette mise en forme peut être réalisée par pressage en même temps que l'élimination du liquide volatil.

Le procédé de préparation de l'invention comprend une étape d'élimination du liquide volatil encore appelé séchage, dont l'objectif est d'éliminer de façon substantielle le liquide volatil pour solidifier la composition.

L'élimination du liquide volatil peut être réalisée avec des moyens mécaniques tels que l'aspiration, ou des moyens thermiques tels que la chaleur.

L'homme du métier saura adapter la durée de séchage nécessaire pour optimiser l'élimination du liquide volatil. La pâte peut comprendre une proportion de liquide volatil plus ou moins élevée en fonction de la durée et des moyens d'élimination utilisés.

La composition à base de poudres obtenue par le procédé de l'invention peut comprendre des traces de liquide volatil utilisé pour la préparation de la pâte. Cette proportion est de préférence inférieure à 2 % en poids, de préférence encore inférieure à 1% en poids par rapport au poids total de la composition. On cherche généralement à réduire au maximum la proportion de liquide volatil résiduel dans la composition finale. La composition de l'invention à base de poudres peut être qualifiée de poudre « sèche », compte tenu de la très faible quantité résiduelle de liquide volatil utilisé pour sa préparation.

Le séchage peut comprendre une étape consistant en l'exposition de la pâte à un courant d'air dont la température est de l'ordre de 20 à 25°C, à un rayonnement infra-rouge, à un rayonnement micro-onde, ou dans un four.

Le séchage peut également comprendre une étape de chauffage dans un four réglé à une température allant de 30 à 70 °C, de préférence allant de 40 à 60 °C. Habituellement, le séchage de la composition peut être complet au bout d'une durée allant de quatre à dix heures dans ces conditions. La pression régnant dans le four est de préférence la pression atmosphérique, et le taux d'humidité dans le four est de préférence contrôlé entre 70 et 90% d'humidité relative.

Le séchage peut également être mis en oeuvre par pressage, par aspiration, par gravité en retournant les moules contenant la pâte, par centrifugation, ou par application d'un matériau absorbant formant buvard sur la surface libre de la pâte qui a été versée dans les moules.

Selon un mode de réalisation, le séchage comprend une étape de séchage par pressage et aspiration du liquide volatil sous vide, à température ambiante (environ 25°C). Le pressage est réalisé grâce à une empreinte qui entre en contact avec la pâte et qui comprime la composition entre le fond du moule et sa surface pendant l'aspiration.

L'extrémité de l'empreinte ou le fond du moule est perforé pour permettre l'extraction du liquide volatil hors de la pâte pendant l'aspiration.

L'aspiration est généralement réalisée en deux étapes, une première série de pressages réalisés grâce à une première empreinte, chaque pressage étant effectué à une pression d'aspiration P1 pendant une durée T1 pour amorcer l'élimination du liquide, suivie d'une deuxième série de pressages réalisés grâce une deuxième empreinte, identique ou différente de la première empreinte, chaque pressage de ladite deuxième série étant effectué à une pression d'aspiration P2 supérieure à P1 pendant une durée T2 supérieure à T1 de sorte à poursuivre l'aspiration du liquide jusqu'à obtenir un état de surface fidèle à ladite deuxième empreinte. Cette aspiration, réalisée de préférence en deux étapes, permet d'améliorer l'état de surface de la composition cosmétique en évitant la formation de fissures ou d'irrégularités susceptibles de se produire pendant l'élimination du liquide volatil. Une série de pressages au sens de l'invention comprend au moins un pressage au moyen d'une presse.

L'un des objectifs de l'opération de pressage - aspiration décrite ci-dessus est une mise en forme de la poudre, sans être pour autant un compactage. Les pressions utilisées sont dès lors relativement douces. La pression d'aspiration P1 est de préférence de l'ordre de 0,05 à 0,3 MPa, tandis que la pression d'aspiration P2 est de préférence de l'ordre de 0,3 à 0,8 MPa.

On réalise par exemple:
- une première étape comprenant au moins deux presses d'une durée respective de 1 à 10 secondes, typiquement 5 secondes, à une première pression, généralement de l'ordre de 0,05 à 0,3 MPa, typiquement de l'ordre de 0,1 MPa, pour commencer l'élimination du liquide volatil, puis
- une deuxième étape comprenant au moins une presse d'une durée de 5 à 20 secondes, typiquement 10 secondes, à une seconde pression généralement de l'ordre de 0,3 à 0,8 MPa, typiquement de l'ordre de 0,5 MPa.

Dans ce mode de réalisation, un matériau poreux peut être déposé à la surface libre de la pâte dans chaque moule, ou être placé sur l'empreinte qui sert de presse afin de retenir la poudre dans le moule pendant l'aspiration. Ce matériau poreux est par exemple une toile, une trame ou une feuille absorbante. Il est ôté de la composition une fois l'aspiration terminée.

L'élimination du liquide volatil peut également comprendre la combinaison des moyens décrits précédemment. Ainsi, conformément à une variante du procédé de l'invention, l'élimination du liquide volatil contenu dans la pâte comprend une élimination mécanique suivie d'une élimination thermique. Le procédé peut comprendre par exemple deux étapes successives : le séchage partiel de la pâte à l'aide de moyens d'élimination mécaniques pour obtenir une poudre humide, et le séchage de la poudre humide par des moyens thermiques. L'élimination mécanique peut être réalisée par aspiration sous presse, et l'élimination thermique peut être réalisée par exemple par un séchage en étuve, de préférence à une température inférieure à 70°C, de préférence encore inférieure à 60°C.

Selon un premier mode de réalisation préféré, les compositions de l'invention sont préparées par un procédé qui comprend :
- la préparation de la pâte,
- l'introduction de la pâte dans un moule, par exemple au moyen d'une pompe et d'une unité d'injection qui comprend avantageusement des buses permettant d'injecter la pâte par le dessous ou le dessus des moules,
- le dépôt d'un matériau poreux à la surface libre de la pâte,
- la mise sous vide des moules remplis et l'élimination par aspiration du liquide volatil présent dans la pâte, de préférence et maintenant la pâte sous presse pendant l'aspiration, et
- le retour à la pression atmosphérique.

On peut ensuite faire sécher les moules au four à pression atmosphérique avec un taux d'humidité contrôlé, selon les conditions de séchage en four décrites précédemment.

Selon un deuxième mode de réalisation préféré, la pâte est étalée sur une plaque, puis séchée en étuve à un taux d'humidité contrôlé. L'élimination du liquide volatil peut être suivie d'un tamisage. La poudre libre obtenue peut être proposée à la vente après avoir été placée dans des conditionnements, tels que des flacons dotés d'un applicateur solidaire du couvercle ou du bouchon. Selon ce procédé, on pourra préparer des poudres libres pour le visage (fond de teint poudre, blush ou fard à joues) ou pour les yeux (fards à paupières).

Selon un troisième mode de réalisation du procédé de l'invention, on procède à l'élimination du liquide volatil dans la pâte pour obtenir une poudre sèche, qui est ensuite tamisée puis compactée. Le compactage est réalisé dans les conditions classiques connues de l'homme du métier, soit directement dans un conditionnement dans lequel la poudre sèche aura été versée, soit dans une coupelle qui sera ensuite placée dans un boitier.

La composition une fois séchée dans le moule peut être démoulée et manipulée pour être placée dans le conditionnement destinée à la vente. Le moule peut aussi être un godet ou une coupelle qui sera directement encliqueté ou placé dans le conditionnement sans qu'il soit nécessaire de démouler la composition.

Les compositions de l'invention permettent de résoudre les problèmes techniques énoncés. La présente invention convient particulièrement pour la préparation de compositions cosmétiques destinées au soin ou maquillage du corps et/ou du visage.

Le procédé de l'invention permet de préparer une poudre homogène dans laquelle les différents composants sont bien dispersés. La dispersion des différents ingrédients dans la poudre obtenue selon ce procédé est en effet meilleure que celle des poudres obtenues par un procédé de l'art antérieur qui utilise la voie sèche.

Les compositions de l'invention présentent en particulier de très bonnes performances en terme notamment de résistance à l'eau et de tenue sur la peau du visage, en particulier des paupières.

Ces compositions sont donc particulièrement adaptées à la préparation de compositions cosmétiques de soin et/ de maquillage du corps ou du visage.

La présente invention décrit donc donc une méthode de soin cosmétique et/ou de maquillage comprenant l'application de la composition à base de poudres sur au moins une partie de la peau.

D'autres buts, caractéristiques et avantages de l'invention apparaîtront clairement à l'homme de l'art suite à la lecture de la description explicative qui fait référence à des exemples qui sont donnés seulement à titre d'illustration et qui ne sauraient en aucune façon limiter la portée de l'invention.

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité. Ainsi, chaque exemple a une portée générale.

D'autre part, dans les exemples, tous les pourcentages sont donnés en poids, sauf indication contraire, et la température est exprimée en degré Celsius sauf indication contraire, et la pression est la pression atmosphérique, sauf indication contraire. Les noms des produits chimiques correspondent à leurs dénomination INCI ou à leur nom chimique selon les règles de nomenclature de la IUPAC.

### Exemple 1: Fard à paupières en poudre compacte

On a fabriqué des fards à paupières compactes de composition suivante :

| Phase A | |
|---|---|
| TALC | Qsp 100 |
| ACIDE SORBIQUE | 0,3 |
| OXYDE DE FER CI 77499 | 13,2 |
| | |

| Phase B | |
|---|---|
| MICA/SILICA/DIOXYDE DE TITANE CI 77891 | 5,0 |
| MICA/OXYDE DE FER CI 77491 | 7,0 |
| Phase C | |
| PENTYLENE GLYCOL | 2,5 |
| PHENOXYETHANOL | 0,9 |
| Ammoniun Acryloyldimethyltaurate / VP | |
| Copolymer | 0,2 |
| OCTYLDODECYL STEAROLYL STEARATE | 20,0 |

### Préparation de la pâte

Les ingrédients de la phase A ont été mélangés dans un mélangeur de marque Lödige ou Waring pendant 10 à 30 minutes. Les ingrédients de la phase B ont été ajoutés dans le mélangeur et mélangés à la phase A pendant au moins 5 minutes.

Dans un Rayneri, on a mélangé les ingrédients de la phase C à de l'eau purifiée pour préparer une émulsion dans laquelle l'eau représente 50% en poids du poids de l'émulsion.

Le mélange des phases A et B a été versé sous agitation dans le Rayneri contenant l'émulsion. La pâte a été malaxée pendant 10 à 30 minutes. Si nécessaire, de l'eau a été ajoutée pour ajuster la consistance de la pâte. La pâte a été lissée pendant 5 à 10 minutes supplémentaires si nécessaire, pour s'assurer de l'homogénéité de la pâte.

### Moulage des poudres

La pâte a été placée dans un pétrin et façonnée en forme de boudins, puis découpée pour être placée dans des godets. Le séchage a été réalisé par aspiration du liquide volatil sous vide en appliquant successivement:
- deux presses, d'une durée de 5 secondes, à une pression de 0,1 MPa, pour commencer l'aspiration du liquide volatil, puis
- une presse, d'une durée de 10 secondes, à une pression de 0,5 MPa.

Une toile a été placée sur la tête de chaque empreinte pour qu'elle n'entre pas directement en contact avec la pâte.

Les godets remplis et pressés ont ensuite été placés dans un four réglé à une température de 50°C, pendant une durée de 5 heures, à la pression atmosphérique, et à un taux d'humidité de 80%.

### Test de résistance aux chocs mécaniques

Des tests de chute ont été réalisés sur les poudres compactes préparées précédemment selon le protocole suivant. On a fait varier le pourcentage de copolymère bloc (Ammoniun Acryloyldimethyltaurate / VP Copolymer) en ajustant le pourcentage de liant OCTYLDODECYL STEAROLYL STEARATE, sans faire varier les proportions des autres ingrédients de la formule.

Le protocole qui a été suivi pour définir la résistance à la chute des poudres compactes consiste à
- se placer au-dessus d'une paillasse et à tenir une règle graduée de 30 cm verticalement d'une main.
- Tenir de l'autre main le godet horizontalement - le fond du godet vers le bas - au-dessus de la paillasse.
- Lâcher le godet et recommencer l'opération jusqu'à l'apparition de la première fêlure ou cassure, et noter le nombre de chutes effectuées.
- Répétez l'opération sur au moins deux ou trois godets, puis effectuer la moyenne des résultats.

| | Nombre de chutes | | |
|---|---|---|---|
| % Copolymère bloc | 24 heures° | 21 jours° | Moyenne |
| 0% | 2,3 | 4,3,3 | 3 |
| 0,1% | 7,7 | 4,2,5 | 5 |
| 0,2% | 9,10 | 9,17,10 | 11 |
| 0,4% | 15,44 | 19*,20*,22 | 24 |

| | | | |
|---|---|---|---|
| *décollement de la plaque complète ^{∘}après le moulage des poudres | | | |

Le copolymère bloc améliore très nettement la résistance aux chocs mécaniques des poudres compactes.

### Exemple 2: Fard à paupières en poudre compacte

On a fabriqué des fards à paupières compactes de composition suivante:

| Phase A | |
|---|---|
| TALC | Qsp 100 |
| ACIDE SORBIQUE | 0,3 |
| OXYDE DE FER CI 77499 | 1,0 |
| MYRISTATE DE MAGNESIUM | 2,0 |
| | |

| Phase B | |
|---|---|
| MICA/DIOXYDE DE TITANE CI 77891 | 30 |
| SILICE/ALUMINE | 5,0 |
| MICA/DIOXYDE DE TITANE CI 77891/ FERROCYANURE FERRIQUE | 0,5 |
| MICA/OXYDE DE FER CI 77491 | 0,5 |
| | |

| Phase C | |
|---|---|
| PENTYLENE GLYCOL | 2,5 |
| PHENOXYETHANOL | 0,9 |
| Ammoniun Acryloyldimethyltaurate / VP Copolymer | 0,2 |
| OCTYLDODECYL STEAROLYL STEARATE | 22,0 |

### Préparation de la pâte

Elle a été réalisée suivant le protocole de l'exemple 1.

### Moulage des poudres

Il a été réalisé dans les mêmes conditions que celles de l'exemple 1.

### Test de résistance aux chocs mécaniques

Il a été réalisé dans les mêmes conditions que celles de l'exemple 1.

| | Nombre de chutes | | |
|---|---|---|---|
| % Copolymère bloc | 24 heures° | 21 jours° | Moyenne |
| 0% | 2,2 | 4,4,7 | 3 |
| 0,1% | 10,8 | 8*,8*,10 | 8 |
| 0,2% | 6,7 | 9*,11,10 | 8 |
| 0,4% | 14*,14* | 13*,16*,15* | 14 |

| | | | |
|---|---|---|---|
| *décollement de la plaque complète ^{∘}après le moulage des poudres | | | |

Le copolymère bloc améliore très nettement la résistance aux chocs mécaniques des poudres compactes.

### Exempté 3 : Fard à paupières en poudre compacte

On a fabriqué des fards à paupières compactes de composition suivante :

| Phase A | |
|---|---|
| TALC | Qsp 100 |
| ACIDE SORBIQUE | 0,3 |
| OXYDE DE FER CI 77499 | 25 |

| Phase B | |
|---|---|
| FERROCYANURE FERRIQUE/MICA/ OXYDE DE FER CI 77491/ DIOXYDE DE TITANE CI 77891 | 5,0 |
| OXYDE DE FER CI 77499/MICA/ DIOXYDE DE TITANE CI 77891 | 25,0 |
| OXYDE DE FER CI 77491/MICA | 5,0 |

| Phase C | |
|---|---|
| PENTYLENE GLYCOL | 2,5 |
| PHENOXYETHANOL | 0,9 |
| Ammoniun Acryloyldimethyltaurate / VP Copolymer | 0,2 |
| OCTYLDODECYL STEAROLYL STEARATE | 20,0 |

### Préparation de la pâte

Elle a été réalisée suivant le protocole de l'exemple 1.

### Moulage des poudres

Il a été réalisé dans les mêmes conditions que celles de l'exemple 1.

### Test de résistance aux chocs mécaniques

Il a été réalisé dans les mêmes conditions que celles de l'exemple 1.

| | Nombre de chutes | | |
|---|---|---|---|
| % Copolymère bloc | 24 heures° | 21 jours° | Moyenne |
| 0% | 3, 5 | 2, 6, 6 | 4 |
| 0,1% | 5, 8* | 7, 5, 7 | 6 |
| 0,2% | 6, 16*, 20 | 17*, 15, 15 | 17 |
| 0,4% | 15*, 42 | 22*, 14, 20* | 22 |

| | | | |
|---|---|---|---|
| *décollement de la plaque complète ^{∘}après le moulage des poudres | | | |

Le copolymère bloc améliore très nettement la résistance aux chocs mécaniques des poudres compactes.

### Exemple 4 : Fond de Teint en poudre compacte

On a fabriqué un fond de teint compact de composition suivante :

| Phase A | |
|---|---|
| TALC | Qsp 100 |
| EDTA TETRASODIQUE | 0,2 |
| OXYDE DE FER/DIMETHICONE | 4,3 |
| DIOXYDE DE TITANE/DIMETHICONE | 3,7 |

| Phase B | |
|---|---|
| HDI/TRIMETHYLOL HEXYL LACTONE CROSSPOLYMER (PLASTIC POWDER® D-400) | 4,6 |
| METHYL METHACRYLATE CROSSPOLYMER (COVABEAD® LH 170) | 23,1 |

| Phase C | |
|---|---|
| PHENOXYETHANOL | 0,4 |
| GLYCEROL | 7,0 |
| Ammoniun Acryloyldimethyltaurate / VP Copolymer | 0,4 |

### Préparation de la pâte

Elle a été réalisée selon le protocole de l'exemple 1.

### Moulage des poudres

La pâte a été placée dans un pétrin et façonnée en forme de boudins, qui ont été placés dans des godets. Le séchage a été réalisé par aspiration du liquide volatil sous vide en appliquant successivement:
- une presse, d'une durée de 5 secondes, à une pression de 0,1 MPa, pour commencer l'aspiration du liquide volatil, puis
- une presse, d'une durée de 10 secondes, à une pression de 0,5 MPa.

Une toile a été placée sur la tête de chaque empreinte pour qu'elle n'entre pas directement en contact avec la pâte.

Les godets remplis et pressés ont ensuite été placés dans un four réglé à une température de 50°C, pendant une durée de 5 heures, à la pression atmosphérique, et à un taux d'humidité de 80%.

### Exemple 5 : Fard à paupières en poudre compacte

On a préparé un fard à paupières compacte en fabriquant une pâte de composition suivante :

| Phase A | |
|---|---|
| TALC | Qsp 100 |
| MICA/LAUROYL LYSINE | 30,0 |
| STEARATE DE MAGNESIUM | 1,9 |
| NYLON-12 | 4,9 |
| MANGANESE VIOLET | 3,0 |
| OXYDES DE FER | 21,9 |
| DIOXYDE DE TITANE | 0,5 |

| Phase B | |
|---|---|
| PHENOXYETHANOL | 0,8 |
| POLYISOBUTENE HYDROGENE | 4,5 |
| Ammoniun Acryloyldimethyltaurate / VP Copolymer | 0,1 |

La phase A a été mélangée dans un appareil Lödige pendant 10 à 15 minutes. La phase B a été préparée et mélangée à la phase A selon le protocole décrit pour la phase C dans l'exemple 1. L'eau représentait 43 % en poids du poids de la pâte.

La pâte a été étalée en couche sur une plaque et placée en étuve réglée à une température de 50°C, pendant une durée de 5 heures, à la pression atmosphérique, et à un taux d'humidité de 80%.

La poudre obtenue a été tamisée, placée dans des godets puis compactée.

### Exemple 6 : Fard à paupières en poudre libre

On a préparé un fard à paupières sous forme de poudre libre de composition suivante :

| Phase A | |
|---|---|
| TALC | Qsp 100 |
| MICA/LAUROYL LYSINE. | 30,3 |
| STEARATE DE MAGNESIUM | 1,9 |
| NYLON-12 | 5,0 |
| MANGANESE VIOLET | 3,0 |
| OXYDES DE FER | 22,2 |
| MICA/DIOXYDE DE TITANE CI 77891/OXYDE DE FER CI 77492 | 0,5 |
| ACIDE SORBIQUE | 0,3 |
| DIOXYDE DE TITANE | 0,5 |

| Phase B | |
|---|---|
| PHENOXYETHANOL | 1,1 |
| PENTYLENE GLYCOL | 2,5 |
| Ammoniun Acryloyldimethyltaurate / VP Copolymer | 0,05 |

La pâte a été préparée selon le protocole de l'exemple 5. La poudre obtenue a ensuite été tamisée et conditionnée.

## Revendications

1. Composition cosmétique solide sous forme de poudre libre ou compacte, comprenant :
(a) de 70 à 99% en poids d'au moins une phase solide particulaire,
(b) de 0,1 à 50% en poids d'au moins une phase liquide non volatile, et
(c) de 0,01 à 4% en poids d'au moins d'un copolymère bloc obtenu à partir d'un premier monomère de l'acide acrylamido-2-méthylpropane-sulfonique ou d'un de ses sels, et d'un deuxième monomère vinylique ayant une chaine latérale azotée.

2. Composition selon la revendication 1, **caractérisée en ce qu'**elle comprend de 75 à 99% d'au moins une phase solide particulaire.

3. Composition selon les revendications 1 ou 2, **caractérisée en ce que** la phase solide particulaire contient des charges sphériques, et que ces charges représentent de 25 à 35% en poids de la composition.

4. Composition selon l'une quelconque des revendications précédentes, **caractérisée en ce qu'**elle contient de 5 à 25% en poids d'au moins une phase liquide non volatile.

5. Composition l'une quelconque des revendications précédentes, **caractérisée en ce que** la phase liquide non volatile contient au moins un liquide choisi parmi une huile, un alcool choisi parmi les polyols C3-C5, tel que le glycérol, les éthers de glycol, et leurs mélanges.

6. Composition selon l'une quelconque des revendications précédentes, **caractérisée en ce que** le deuxième monomère est la N-vinylpyrrolidone.

7. Composition selon l'une quelconque des revendications précédentes, **caractérisée en ce qu'**elle contient moins de 0,1% en poids de corps gras.

8. Procédé de fabrication d'une composition selon l'une quelconque des revendications précédentes **caractérisé en ce qu'**il comprend:
(i) le mélange de la phase solide particulaire, de la phase liquide non volatile, du copolymère bloc et d'un liquide volatil pour obtenir une pâte,
(ii) la mise en moule de la pâte,
(ii) l'élimination au moins partielle dudit liquide volatil présent dans la pâte.

9. Procédé selon la revendication 8, le liquide volatil représente de 20 à 70% en poids, de préférence de 30 à 60% en poids, du poids de la pâte.

10. Procédé selon l'une quelconque des revendications 8 et 9, **caractérisé en ce que** le liquide volatil contient une quantité majoritaire d'eau.

11. Procédé selon l'une quelconque des revendications 8 à 10, **caractérisé en ce que** la composition n'est pas démoulée après l'élimination du liquide volatil.

12. Procédé selon l'une quelconque des revendications 8 à 11, **caractérisé en ce que** l'élimination du liquide volatil est effectuée en deux étapes successives: une élimination mécanique pour obtenir une poudre humide, et le séchage de la poudre humide par un procédé thermique.

13. Procédé selon l'une quelconque des revendications 8 à 12, **caractérisé en ce que** l'étape d'élimination du liquide volatil est réalisée par aspiration sous presse, puis par séchage en étuve à une température inférieure à 60°C.

14. Procédé selon la revendication 13, **caractérisé en ce que** l'aspiration sous presse est réalisée en deux étapes, une première série de pressages réalisés grâce à une première empreinte, chaque pressage étant effectué à une pression P1 comprise entre 0,05 et 0,3 MPa pendant une durée T1 pour amorcer l'élimination du liquide, suivie d'une deuxième série de pressages réalisés grâce à une deuxième empreinte, chaque pressage de ladite deuxième série étant effectué à une pression P2 comprise entre 0,3 et 0,8 MPa pendant une durée T2 supérieure à T1 de sorte à poursuivre l'élimination du liquide jusqu'à obtenir un état de surface fidèle à la deuxième empreinte.

15. Procédé de fabrication d'une composition à base de poudres, lequel procédé comprend :
(i) la mise en dispersion dans l'eau d'une phase solide particulaire, d'une phase liquide non volatile, et d'un copolymère bloc ionique obtenu à partir d'un premier monomère d'un sel de l'acide acrylamido-2-alkyl-sulfonique et d'un deuxième monomère, pour obtenir une pâte,
(ii) la mise en moule, et
(iii) l'élimination de l'eau présente dans la pâte par aspiration sous vide suivie d'un séchage à une température inférieure à 70°C.

16. Procédé selon la revendication précédente, **caractérisé en ce que** le deuxième monomère est choisi parmi l'acide (méth)acrylique, les (méth)acrylate, les (méth)acrylamides, les sels de (méth)acrylate et la vinylpyrolidone.

17. Procédé selon les revendications 15 ou 16, **caractérisée en ce que** le copolymère bloc est le Sodium Acrylate / Sodium Acryloyldimethyltaurate Copolymer, le Sodium Acryloyldimethyltaurate / VP Crosspolymer, le Acrylamide / Sodium Acryloyldimethyltaurate Copolymer, ou le Ammonium Acryloyldimethyltaurate / VP Copolymer.

## Patentansprüche

1. Feste kosmetische Zusammensetzung in loser oder kompakter Pulverform, umfassend:
(a) 70 bis 99 Gew.-% mindestens einer teilchenförmigen festen Phase,
(b) 0,1 bis 50 Gew.-% mindestens einer nichtflüchtigen flüssigen Phase und
(c) 0,01 bis 4 Gew.-% mindestens eines Blockcopolymers, das aus einem ersten Monomer von Acrylamido-2-methylpropan-sulfonsäure oder einem Salz davon und einem zweiten Monomer aus Vinyl mit einer Stickstoffseitenkette erhalten wird.

2. Zusammensetzung gemäß Anspruch 1, **dadurch gekennzeichnet, dass** sie 75 bis 99% mindestens einer teilchenförmigen festen Phase umfasst.

3. Zusammensetzung gemäß Anspruch 1 oder 2, **dadurch gekennzeichnet, dass** die teilchenförmige feste Phase kugelförmige Ladungen enthält und dass diese Ladungen 25 bis 35 Gew.-% der Zusammensetzung ausmachen.

4. Zusammensetzung gemäß einem der vorangehenden Ansprüche, **dadurch gekennzeichnet, dass** sie 5 bis 25 Gew.-% mindestens einer nichtflüchtigen flüssigen Phase enthält.

5. Zusammensetzung gemäß einem der vorangehenden Ansprüche, **dadurch gekennzeichnet, dass** die nichtflüchtige flüssige Phase mindestens eine Flüssigkeit, ausgewählt aus einem Öl, einem Alkohol, ausgewählt aus C3-C5-Polyolen, wie Glycerin, Glykolethern, und Mischungen davon, enthält.

6. Zusammensetzung gemäß einem der vorangehenden Ansprüche, **dadurch gekennzeichnet, dass** es sich bei dem zweiten Monomer um N-Vinylpyrrolidon handelt.

7. Zusammensetzung gemäß einem der vorangehenden Ansprüche, **dadurch gekennzeichnet, dass** sie weniger als 0,1 Gew.% Fett enthält.

8. Verfahren zur Herstellung einer Zusammensetzung gemäß einem der vorangehenden Ansprüche, **dadurch gekennzeichnet, dass** es umfasst:
(i) Mischen der teilchenförmigen festen Phase, der nichtflüchtigen flüssigen Phase, des Blockcopolymers und einer flüchtigen Flüssigkeit, um eine Paste zu erhalten,
(ii) Einfüllen der Paste in die Form,
(ii) zumindest teilweises Entfernen der flüchtigen Flüssigkeit, die in der Paste vorhanden ist.

9. Verfahren gemäß Anspruch 8, wobei die flüchtige Flüssigkeit 20 bis 70 Gew.-%, vorzugsweise 30 bis 60 Gew.-%, des Gewichts der Paste ausmacht.

10. Verfahren gemäß einem der Ansprüche 8 und 9, **dadurch gekennzeichnet, dass** die flüchtige Flüssigkeit eine überwiegende Menge Wasser enthält.

11. Verfahren gemäß einem der Ansprüche 8 bis 10, **dadurch gekennzeichnet, dass** die Zusammensetzung nach Entfernen der flüchtigen Flüssigkeit nicht aus der Form herausgenommen wird.

12. Verfahren gemäß einem der Ansprüche 8 bis 11, **dadurch gekennzeichnet, dass** das Entfernen der flüchtigen Flüssigkeit in zwei aufeinander folgenden Schritten durchgeführt wird: mechanisches Entfernen, um ein feuchtes Pulver zu erhalten, und Trocknen des feuchten Pulvers durch ein thermisches Verfahren.

13. Verfahren gemäß einem der Ansprüche 8 bis 12, **dadurch gekennzeichnet, dass** der Schritt des Entfernens der flüchtigen Flüssigkeit durch Ansaugen unter Druck und anschließendes Trocknen in einem Ofen bei einer Temperatur unter 60°C durchgeführt wird.

14. Verfahren gemäß Anspruch 13, **dadurch gekennzeichnet, dass** das Ansaugen unter der Druck in zwei Schritten durchgeführt wird, wobei eine erste Reihe von Druckvorgängen mittels eines ersten Abdrucks durchgeführt wird, wobei jeder Druckvorgang bei einem Druck P1 zwischen 0,05 und 0,3 MPa für eine Zeitspanne T1 ausgeführt wird, um das Entfernen der Flüssigkeit zu initiieren, gefolgt von einer zweiten Reihe von Druckvorgängen, die mittels eines zweiten Abdrucks durchgeführt wird, wobei jeder Druckvorgang der zweiten Reihe bei einem Druck P2 zwischen 0,3 und 0,8 MPa für eine Zeitspanne T2, die größer als T1 ist, ausgeführt wird, um das Entfernen der Flüssigkeit so lange fortzusetzen, bis ein Zustand der Oberfläche erreicht ist, der dem zweiten Abdruck entspricht.

15. Verfahren zur Herstellung einer Pulverzusammensetzung, wobei das Verfahren umfasst:
(i) Diespergieren einer teilchenförmigen festen Phase, einer nichtflüchtigen flüssigen Phase und eines ionischen Blockcopolymers, das aus einem ersten Monomer eines Salzes einer Acrylamido-2-alkyl-sulfonsäure und einem zweiten Monomer erhalten wird, in Wasser, um eine Paste zu erhalten,
(ii) Einfüllen in die Form,
(iii) Entfernen des Wassers aus der Paste durch Vakuumansaugen und anschließendes Trocknen bei einer Temperatur unter 70°C.

16. Verfahren gemäß dem vorangehenden Anspruch, **dadurch gekennzeichnet, dass** das zweite Monomer aus (Meth)acrylsäure, (Meth)acrylat, (Meth)acrylamid, (Meth)acrylatsalzen und Vinylpyrolidon ausgewählt ist.

17. Verfahren gemäß den Ansprüchen 15 oder 16, **dadurch gekennzeichnet, dass** es sich bei dem Blockcopolymer um Natriumacrylat / Natriumacryloyldimethyltaurat-Copolymer, Natriumacryloyldimethyltaurat / VP-Crosspolymer, Acrylamid / Natriumacryloyldimethyltaurat-Copolymer oder Ammoniumacryloyldimethyltaurat / VP-Copolymer handelt.

## Claims

1. Solid cosmetic composition, in the form of a loose or compact powder, comprising:
(a) from 70% to 99% by weight of at least one particulate solid phase,
(b) from 0.1% to 50% by weight of at least one non-volatile liquid phase, and
(c) from 0.01% to 4% by weight of at least one block copolymer obtained from a first monomer of 2-acrylamidomethylpropanesulphonic acid or a salt thereof, and from a vinyl second monomer having a nitrogenous side chain.

2. Composition according to Claim 1, **characterized in that** it comprises from 75% to 99% of at least one particulate solid phase.

3. Composition according to Claim 1 or 2, **characterized in that** the particulate solid phase contains spherical fillers, and that these fillers represent from 25% to 35% by weight of the composition.

4. Composition according to either one of the preceding claims, **characterized in that** it contains from 5% to 25% by weight of at least one non-volatile liquid phase.

5. Composition according to any one of the preceding claims, **characterized in that** the non-volatile liquid phase contains at least one liquid chosen from an oil, an alcohol chosen from C3-C5 polyols, such as glycerol, glycol ethers, and mixtures thereof.

6. Composition according to any one of the preceding claims, **characterized in that** the second monomer is N-vinylpyrrolidone.

7. Composition according to any one of the preceding claims, **characterized in that** it contains less than 0.1% by weight of fatty substances.

8. Process for manufacturing a composition according to any one of the preceding claims, **characterized in that** it comprises:
(i) mixing the particulate solid phase, the non-volatile liquid phase, the block copolymer and a volatile liquid so as to obtain a slurry,
(ii) placing the slurry in a mould,
(iii) at least partially removing said volatile liquid present in the slurry.

9. Process according to Claim 8, **characterized in that** the volatile liquid represents from 20% to 70% by weight, preferably from 30% to 60% by weight, of the weight of the slurry.

10. Process according to either one of Claims 8 and 9, **characterized in that** the volatile liquid contains a majority amount of water.

11. Process according to any one of Claims 8 to 10, **characterized in that** the composition is not demoulded after the removal of the volatile liquid.

12. Process according to any one of Claims 8 to 11, **characterized in that** the removal of the volatile liquid is carried out in two successive steps: a mechanical removal so as to obtain a wet powder, and drying of the wet powder by means of a thermal process.

13. Process according to any one of Claims 8 to 12, **characterized in that** the step of removing the volatile liquid is carried out by suctioning off under a press, and then by drying in an oven at a temperature of less than 60°C.

14. Process according to any one of Claims 8 to 13, **characterized in that** the suctioning off under a press is carried out in two steps, a first series of pressings carried out by means of a first stamp, each pressing being performed at a pressure P1 of between 0.05 and 0.3 MPa for a time T1 so as to initiate the removal of the liquid, followed by a second series of pressings carried out by means of a second stamp, each pressing of said second series being performed at a pressure P2 of between 0.3 and 0.8 MPa for a time T2 greater than T1, in such a way as to continue the removal of the liquid until a surface finish faithful to the second stamp is obtained.

15. Process for manufacturing a powder-based composition, which process comprises:
(i) dispersing, in water, a particulate solid phase, a non-volatile liquid phase, and an ionic block copolymer obtained from a first monomer of a 2-acrylamidoalkylsulfonic acid salt and from a second monomer, so as to obtain a slurry,
(ii) placing it in a mould, and
(iii) removing the water present in the slurry by suctioning off under vacuum, followed by drying at a temperature of less than 70°C.

16. Process according to the preceding claim, **characterized in that** the second monomer is chosen from (meth)acrylic acid, (meth)acrylates, (meth)acrylamides, (meth)acrylate salts and vinylpyrrolidone.

17. Process according to Claim 15 or 16, **characterized in that** the block copolymer is Sodium Acrylate/Sodium Acryloyldimethyltaurate Copolymer, Sodium Acryloyldimethyltaurate/VP Crosspolymer, Acrylamide/Sodium Acryloyldimethyltaurate Copolymer, or Ammonium Acryloyldimethyltaurate/VP Copolymer.
